# EUROPEAN PATENT SPECIFICATION

(11) **EP 1 527 704 B1**
(45) Date of publication and mention of the grant of the patent: **02.07.2008**
(21) Application number: 04252028.8
(22) Date of filing: 05.04.2004
(51) Int. Cl.: A41D 19/00

(54) **Glove with internal carrier medium and method for producing the same**
Handschuh mit inneres Träger und ihr Herstellungsverfahren
Gant avec support interne et son procédé de production

(30) Priority: 30.10.2003 JP 2003370837
(43) Date of publication of application: 04.05.2005
(73) Proprietor: Yugengaisha Panmedica, Saitama-shi, Saitama-ken (JP)
(72) Inventor: Kitamura, Keikichi, Saitama-shi Saitama-ken (JP)
(74) Representative: Bridge-Butler, Alan James

(56) References cited:
- DE-C- 19 624 245
- GB-A- 2 215 183
- JP-A- 62 006 978
- JP-A- 62 007 000
- US-A- 3 934 062
- PATENT ABSTRACTS OF JAPAN vol. 2000, no. 26, 1 July 2002 (2002-07-01) & JP 2001 254269 A (BERUBIKKU:KK), 21 September 2001 (2001-09-21)
- PATENT ABSTRACTS OF JAPAN vol. 0141, no. 93 (M-0963), 19 April 1990 (1990-04-19) & JP 2 036938 A (MITSUBISHI KASEI CORP), 6 February 1990 (1990-02-06)

## Description

The present invention relates to a glove with an internal carrier medium, which is adapted to be less irritant or inflammatory, for reducing itching and irritation when using a glove made from latex, nitrile, PVC, or the like in a field of medicine, physics and chemistry, industry, or the like as well as a method for producing the same.

In the fields of medicine, physical and chemical research, industry, or the like, gloves tailored to respective applications have heretofore been used. Such gloves are to be put on a hand for the purposes of hygiene, antifungus, hand protection, slip stopper, or the like. Examples of materials to be used in such gloves include latex such as natural rubber, synthetic rubber, or synthetic resin, nitrile rubber, and PVC (polyvinyl chloride).

In use of the above described gloves, allergodermia is a problem in which a nonrubber ingredient such as protein contained in natural rubber or a substance added thereto causes rash, itching, eczema, or the like on the hand; or protein contained in cornstarch powder used as lubricant for smoothly putting on or taking off a glove causes rash, itching, eczema, or the like in combination with sweat inside the glove.

JP Patent Publication (Kokai) No. 6-70942 A (1994) and JP Patent Publication (Kokai) No. 11-81014 A (1999) are conventional examples for preventing itching and irritation caused by wearing such latex gloves. The "Flexible Rubber Product and Method for Producing Them," disclosed in JP Patent Publication (Kokai) No. 6-70942 A (1994), relates to a medical multi-layered glove formed of: a patient contact layer formed from aqueous natural rubber latex emulsion as a film; a wearer contact layer formed from aqueous emulsion containing an acryl copolymer and fluorocarbon telomer resin; and the above patient contact layer and the above wearer contact layer formed from aqueous emulsion containing natural rubber latex, polyurethane latex, poly(acrylamide/acrylic acid), and polyethylene oxide.

The "Latex Glove" shown in JP Patent Publication (Kokai) No. 11-81014 A (1999), relates to a latex glove formed from deproteinized natural rubber, characterized by containing 0.5 parts by weight or more of particulates having a group of -OH per 100 parts by weight of the above natural rubber.

The aim of these two inventions is to reduce protein allergy by reducing a content of protein ingredients in a latex glove.

At the same time, technologies for using phthalocyanine, known as conventional dye or pigment, as a deodorant for fibers such as clothing, bedclothing, and rugs have been developed in new fields, focusing on the deodorant effect.

Moreover, there is a possibility of using phthalocyanine in other applications, because the clinical results recently reported in Japanese Pharmacology & Therapeutics, Life Science Publishing Co., Ltd., issued on April 20,1996, Vol. 24 No. 4, pages 132 to 135, show that phthalocyanine has antipruritic and antiinflammatory effects.

The content of the above Document may be summarized as follows: "Histamine and serotonin are identified in causative agents that generate various symptoms of allergodermia, but it is well known that both are physiologically active amine and have an effect to cause itching and irritation to skin as one of their effects. Two types of general antihistamine and antiserotonin are currently used against these effects: (1) a type that causes antagonistic disorder by binding to histamine, serotonin, and receptors and (2) a type that control release of histamine and serotonin from mast cells. As described above, from clinical experience, we presumed the existence of an antihistamine effect by means of a third mechanism different from the two types and performed the following basic experiments to verify the possibility." As a result, "metal phthalocyanine octacarboxylic acid [metal = Fe, Co] can be said to be fully promising as novel antihistamine and antiserotonin agents having new control mechanisms."

In line with this, JP 2001 254269 in the name of BERUBIKKU KK discloses the imparting of an immobilized metal phthalocyanine derivative to fibrous clothing in order to suppress inflammation and odor. However such a solution is not possible with non fibrous clothing such as latex gloves and the like.

US 3934062 in the name of TIL,LOTSON discloses a method of imparting a phthalate plasticizer to the outside surface of a glove knitted from cotton, in order to improve its constitution. Again, this solution is not possible with non fibrous clothing like latex gloves. In addition, the imparting of a material to the outside of a glove is of no assistance in reducing itching and the like on the inside of the glove.

The present invention has been made in consideration of the above circumstances and has an object to provide a less irritant or inflammatory glove for reducing itching and irritation when using a glove made from latex, nitrile, PVC, or the like in a field of medicine, physics and chemistry, industry, or the like as well as a method for producing the same, in which the reduction is realized not by reducing the content of protein ingredients contained in the latex glove as in prior art, but by utilizing antipruritic and antiinflammatory effects of phthalocyanine.

Therefore, according to a first aspect of the present invention a glove is characterized in that an internal carrier medium is provided inside the glove, and in which the internal carrier medium carries phthalocyanine.

Thus, a less irritant or inflammatory glove is provided which reduces itching and irritation of skin when wearing a glove made from latex, nitrile, PVC, or the like.

In one version of the invention the internal carrier medium can comprise an inner glove knitted with cotton or a synthetic resin yam, and the phthalocyanine can be adhered to the inner glove.

Thus, the inner glove is worn inside a surgical latex glove. Since the inner glove contacts skin during use, phthalocyanine is adhered to the inner glove to allow phthalocyanine to contact skin, resulting in benefit for the wearer by reducing itching and irritation.

The inner glove can be easily produced by a method for adhering the phthalocyanine to the inner glove knitted with cotton or synthetic resin yarn by immersing the inner glove in a phthalocyanine solution, or a method for knitting the inner glove with yam to which the phthalocyanine is adhered by immersing cotton or synthetic resin yarn in the phthalocyanine solution.

In another version of the invention the internal carrier medium can comprise a lubricant comprising cornstarch powder, and the phthalocyanine can be mixed into the lubricant.

The cornstarch powder can be used as lubricant for the time of putting on the glove. The lubricant presents between the inside surface of the tightly fitted latex glove or the like and skin so that a motion of putting on or taking off the glove can be performed smoothly because of the particulate powder. Mixing phthalocyanine in the cornstarch powder to be used as lubricant allows phthalocyanine to contact skin, resulting in benefit for the wearer by reducing itching and irritation.

Alternatively the lubricant can be phthalocyanine catatonically bound to cellulose. Again, the phthalocyanine contacts skin in the same manner as in the above, resulting in benefit for the wearer by reducing itching and irritation.

In another version of the invention the internal carrier medium can comprise a coating material applied to the inside surface of the glove, the coating material being provided for smoothly putting on and taking off the glove, and the phthalocyanine can be mixed into the coating material.

The coating material on the inside surface of the glove, can be provided for smoothly putting on or taking off the glove, and the phthalocyanine contained in the coating material contacts skin, resulting in benefit for the wearer by reducing itching and irritation.

In another version of the invention the internal carrier medium can comprise a filling implanted on an inside surface of the glove, and the phthalocyanine can be attached to said filling.

If phthalocyanine is adhered to an implanted filling provided on the inside surface of the glove, when wearing the glove, the phthalocyanine that is adhered to the implanted filling provided on the glove contacts hand skin, resulting in benefit for the wearer by reducing itching and irritation. Further, a heavy PVC glove, a heavy nitrile glove, a natural rubber heavy glove, or the like is suitable as such a glove that is implanted a filling on the inside surface. The glove is most suitable for an application as an industrial glove.

The invention also includes methods of making the gloves described above.

Therefore, according to a second aspect of the present invention a method of making a glove, characterized in that an inner glove knitted from cotton or a synthetic resin yarn is provided inside the glove, and in which phthalocyanine is adhered to the inner glove, is characterized by comprising the step of immersing the inner glove, or the yarn thereof, in a phthalocyanine solution.

According to a third aspect of the present invention a method of making a glove, characterized in that an inner glove knitted with cotton or a synthetic resin yarn is provided inside the glove, and in which phthalocyanine is adhered to the inner glove, is characterized by comprising the step of immersing the yam of the inner glove in a phthalocyanine solution.

The examples of the present invention will now be described.

Phthalocyanine to be used in the present invention is an organic compound containing metal such as iron, sometimes referred to as metal phthalocyanine, have been used as dye, and known for a person skilled in the art as a substance having a deodorant effect. Among such phthalocyanine, metal phthalocyanine octacarboxylic acid [metal = Fe, Co], for example, can be referred to as one that is expected to have an effect of reducing itching and irritation, which is expected to be a new substance having new antihistamine and antiserotonin effects capable of reducing itching and irritation.

A first embodiment of the invention comprises a glove provided with an inner glove as an internal carrier medium. It should be noted that an inner glove is a thin glove that is worn inside a surgical latex glove. The inner glove is knitted with cotton yam, synthetic resin yam such as polyester, or the like. In this example, phthalocyanine is adhered to the inner glove.

A method for adhering phthalocyanine to the inner glove can be performed by immersing the inner glove knitted with cotton or synthetic resin yarn such as polyester in a phthalocyanine solution so that the phthalocyanine is adhered to the inner glove.

The inner glove can be knitted by yam to which the phthalocyanine is adhered by immersing cotton or synthetic resin yarn in a phthalocyanine solution.

A second embodiment of the invention comprises a glove provided with a lubricant as an internal carrier medium. The glove can be a glove made from natural rubbber, synthetic rubber, or synthetic resin, to which lubricant for the time of putting on the glove is adhered. In this example, cornstarch powder used as such lubricant in admixture with phthalocyanine is adhered to the inside surface of the glove.

Alternatively, phthalocyanine cationically bound to cellulose is used as lubricant when putting on the glove, which is adhered to the inside surface of the glove made from natural rubber, synthetic rubber, or synthetic resin

Further, other than applying lubricant to a reversed glove, a method for adhering the above lubricant to the inside surface of a glove may comprise immersing the glove that is reversed in a phthalocyanine solution followed by drying.

A third embodiment of the invention comprises a glove provided with a coating material as an internal carrier medium. The glove of this example is a glove with an inside surface to which a coating material is applied in order to smoothly put on or take off the glove made from natural rubber, synthetic rubber, or synthetic resin. Urethane, polymers, or the like is usually used as such a coating material. In this example, such a coating material in admixture with phthalocyanine is used. The coating material is coated on the inside surface of the glove made from natural rubber, synthetic rubber, or synthetic resin.

A fourth embodiment of the invention comprises a glove provided with a filling as an internal carrier medium. The glove of this example is one in which phthalocyanine is adhered to a filling provided on the inside surface of the glove. In order to provide the filling on the inside surface of the glove, the rubber surface that is a raw material of the glove is charged with positive ions and provided with a binding material, at the same time, phthalocyanine is impregnated in cotton that is formed to have uniform microlength as a raw material of an implanted filling, which is charged with negative ions to be adhered to the surface charged with positive ions in a condition in which filling is implanted.

In the above explanation, the present invention shows the feature as a less irritant or inflammatory glove. However, the less irritant or inflammatory effect of phthalocyanine can be also expected in its application to a fingerstall that is also worn on a finger within a range in which the above invention can be applied.

## Claims

1. A glove **characterized in that** an internal carrier medium is provided inside the glove, and in which the internal carrier medium carries phthalocyanine.

2. A glove as claimed in Claim 1 in which the internal carrier medium comprises an inner glove knitted with cotton or a synthetic resin yam, and in which the phthalocyanine is adhered to the inner glove.

3. A glove as claimed in Claim 1 in which the internal carrier medium comprises a lubricant comprising cornstarch powder, and in which the phthalocyanine is mixed into the lubricant.

4. A glove as claimed in Claim 1 in which the internal carrier medium comprises a lubricant comprising phthalocyanine catatonically bound to cellulose.

5. A glove as claimed in Claim 1 in which the internal carrier medium comprises a coating material applied to the inside surface of the glove, the coating material being provided for smoothly putting on and taking off the glove, and in which the phthalocyanine is mixed into the coating material.

6. A glove as claimed in Claim 1 in which the internal carrier medium comprises a filling implanted on an inside surface of the glove, and in which the phthalocyanine is attached to said filling.

7. A method of making a glove **characterized in that** an inner glove knitted with cotton or a synthetic resin yam is provided inside the glove, and in which phthalocyanine is adhered to the inner glove, is **characterized by** comprising the step of immersing the inner glove in a phthalocyanine solution.

8. A method of making a glove **characterized in that** an inner glove knitted with cotton or a synthetic resin yam is provided inside the glove, and in which phthalocyanine is adhered to the inner glove, is **characterized by** comprising the step of immersing the yam of the inner glove in a phthalocyanine solution.

## Patentansprüche

1. Handschuh,
**dadurch gekennzeichnet,**
**dass** ein inneres Trägermedium innerhalb des Handschuhs vorgesehen ist und
**dass** das innere Trägermedium Phthalocyanin trägt.

2. Handschuh nach Anspruch 1,
bei dem das innere Trägermedium einen aus Baumwolle oder Kunstfasergarn gewirkten Innenhandschuh aufweist und dass Phthalocyanin auf den Innenhandschuh aufgebracht ist.

3. Handschuh nach Anspruch 1,
bei dem das innere Trägermedium ein Gleitmittel mit Stärkepulver aufweist und
bei dem das Phthalocyanin in das Gleitmittel gemischt ist.

4. Handschuh nach Anspruch 1,
bei dem das innere Trägermedium ein Gleitmittel mit Phthalocyanin aufweist, das kationisch an Zellulose gebunden ist.

5. Handschuh nach Anspruch 1,
bei dem das innere Trägermedium ein auf die Innenoberfläche des Handschuhs aufgebrachtes Deckmaterial aufweist, wobei das Deckmaterial für ein sanftes Anziehen und Ausziehen des Handschuhs vorgesehen ist und das Phthalocyanin in das Deckmaterial gemischt ist.

6. Handschuh nach Anspruch 1,
bei dem das innere Trägermedium eine auf eine Innenoberfläche des Handschuhs eingesetzte Füllung aufweist und bei der das Phthalocyanin an dieser Füllung angebracht ist.

7. Verfahren zur Herstellung eines Handschuhs,
**dadurch gekennzeichnet,**
**dass** ein aus Baumwolle oder einem Kunstharzgarn gewirkter Innenhandschuh innerhalb des Handschuhs vorgesehen ist und Phthalocyanin auf den Innenhandschuh aufgebracht ist, **gekennzeichnet durch** den Schritt des Eintauchens des Innenhandschuhs in eine Phthalocyaninlösung.

8. Verfahren zur Herstellung eines Handschuhs,
**gekennzeichnet dadurch,**
**dass** ein aus Baumwolle oder Kunstharzfasern gewirkter Innenhandschuh innerhalb des Handschuhs vorgesehen ist, und bei dem Phthalocyanin auf den Innenhandschuh aufgebracht wird, **gekennzeichnet durch** den Schritt des Eintauchens des Garns des Innenhandschuhs in eine Phthalocyaninlösung.

## Revendications

1. Gant **caractérisé en ce qu'**un support interne est disposé à l'intérieur du gant et **en ce que** le support interne est porteur de phtalocyanine.

2. Gant selon la revendication 1, dans lequel le support interne comprend un gant interne tricoté avec un fil en coton ou en résine synthétique et dans lequel la phtalocyanine est collée au gant interne.

3. Gant selon la revendication 1, dans lequel le support interne comprend un lubrifiant comprenant de la poudre d'amidon de maïs et dans lequel la phtalocyanine est mélangée dans le lubrifiant.

4. Gant selon la revendication 1, dans lequel le support interne comprend un lubrifiant comprenant de la phtalocyanine liée catatoniquement à de la cellulose.

5. Gant selon la revendication 1, dans lequel le support interne comprend un matériau de revêtement appliqué à la surface intérieure du gant, le matériau de revêtement étant formé pour mettre et retirer le gant sans à-coup et dans lequel la phtalocyanine est mélangée dans le matériau de revêtement.

6. Gant selon la revendication 1, dans lequel le support interne comprend une garniture implantée sur une surface intérieure du gant et dans lequel la phtalocyanine est fixée à ladite garniture.

7. Procédé de fabrication d'un gant **caractérisé en ce qu'**un gant interne tricoté avec un fil de coton de résine synthétique est disposé à l'intérieur du gant et **en ce que** la phtalocyanine est collée au gant interne, **caractérisé en ce qu'**il comprend l'étape consistant à immerger le gant interne dans une solution de phtalocyanine.

8. Procédé de fabrication d'un gant **caractérisé en ce qu'**un gant interne tricoté avec un fil de coton de résine synthétique est disposé à l'intérieur du gant et **en ce que** la phtalocyanine est collée au gant interne, **caractérisé en ce qu'**il comprend l'étape consistant à immerger le fil du gant interne dans une solution de phtalocyanine.
